# EUROPEAN PATENT APPLICATION

(11) **EP 1 529 879 A1**
(43) Date of publication of application: **11.05.2005**
(21) Application number: 03025634.1
(22) Date of filing: 06.11.2003
(51) Int. Cl.: D21H 27/02, A61K 9/00

(54) **Tissue paper having an irregular coloured pattern**

(71) Applicant: SCA Hygiene Products GmbH, 68305 Mannheim (DE)
(72) Inventor: Stein, Anja, 64689 Grasellenbach (DE); Buchalle, Georg, 68309 Mannheim (DE); Klemm, Steffen, 68542 Heddesheim (DE); Sobierajski, Denise, 64653 Lorsch (DE)
(74) Representative: HOFFMANN - EITLE

(57) **Abstract**

The present invention relates to a coloured tissue paper, in particular one where lotion application is visualised by the incorporation of a dye into the lotion. Specifically, it concerns a tissue paper carrying on at least one outer surface a visible irregular pattern of discrete coloured spots. The irregular pattern of discrete coloured spots is obtainable by either applying an aqueous solution of a dye or a lotion containing a dye.

## Description

The present invention relates to a coloured tissue paper, in particular one where lotion application is visualised by the incorporation of a dye into the lotion.

### Technical Background

A standard tissue paper, like toilet tissue or tissue of facial or handkerchief grade is normally white or of a uniform colour. If these tissue papers are treated with cosmetic lotions in order to enhance the softness of the tissue paper and to provide skin care benefits to the user, the lotion does not have a visually distinctive appearance relative to standard tissue. Therefore, it would be advantageous if tissues treated with cosmetic lotions could be visually distinguished from other (untreated) tissues.

CA-A-2,124,240 teaches printing, dying or otherwise colouring an internal surface of one or more plies of a multi-ply tissue paper with a visual indicator. Because of the relative transparency of the thin tissue sheets used in multi-ply tissue products, the colour or pattern imparted to the internal surface is visible through the outer ply, thereby providing an internal indicia that the tissue surface contains a special ingredients.

However, this tissue paper suffers from the disadvantage that special process steps leading to the coloured internal surface have to be used in order to obtain tissue papers wherein the lotion can be visualised. Further, it has been found that the resulting tissue paper visually does not specifically appeal to the consumer.

EP-A2-0 678 614 discloses a continuous process of non-compressively and uniformly applying a liquid saturant throughout a permeable sheet. This liquid saturant may contain certain dyes or colorants at concentrations of less than about 10 wt%.

WO 96/08601 discloses a softening treatment agent for tissue paper products, in particular in form of a lotion, said treatment agent containing 25 to 95 wt% of at least one polyhydroxy compound, such as polyethylene glycol and/or glycerol, 5-75 wt% polysiloxane as well as 0 to 35 wt% water, based on 100 parts by weight of the above mixture. In this documents it is mentioned that this treatment agent may also contain dyes as one example of many other additives.

NO 01/049923, WO 01/049932, WO 01/049933, WO 01/049935, WO 01/049936 and W001/049937 disclose various application techniques for paper treatment agents including lotions. As one among many possible lotion ingredients, dyes are also mentioned.

It is one object of the present invention to provide a tissue paper, in particular lotioned tissue paper having an aesthetically pleasing pattern.

In view of the above, it is one further technical object of the present application to provide in a preferred embodiment lotioned tissue paper wherein the application of lotion is made visible to the user.

According to one.further object, a tissue product shall be provided in a preferred embodiment, which has a discontinuous, but uniform lotion coating providing softness benefits to the user.

### Brief summary of the invention

The above technical objects are solved by a tissue paper, in particular a lotioned tissue paper, carrying on at least one outer surface a visible irregular pattern of discrete coloured spots, in particular lotion spots containing a dye.

Surprisingly, it has been found that consumers of tissue products regard an irregular pattern of coloured spots as particularly aesthetically satisfying. Furthermore, this irregular pattern is more suitable than any other pattern to suggest to the consumer that the tissue paper has been treated with specific softener additives, in the present case preferably a softener lotion. In contrast thereto, regular patterns are typically associated with printing steps where no lotion is applied.

### Brief description of the figure

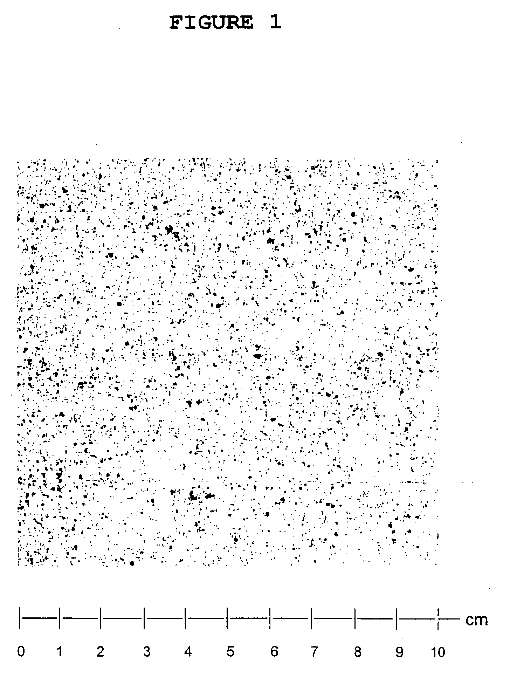

Figure 1 shows a picture of a lotioned tissue paper in accordance with the present invention.

### Detailed description of the present invention

According to a **first embodiment** of the present invention, the tissue paper is lotioned and the lotioned tissue paper of the present invention carries on at least one outer surface, preferably on both outer surfaces, a visible irregular pattern of discrete coloured lotion spots containing a dye.

The term "discrete" means that each lotion spot is clearly visible even though it may overlap with other coloured lotion spots.

Preferably, all lotion spots, if taken together, cover an area of 1-70% of the lotioned surface. The surface coverage is preferably 5-50%, more preferably 7-35%, in particular 10-20%. The term "lotioned surface" refers to all those areas of the lotioned tissue that have been treated with the lotion. As a rule, essentially the entire surface of the tissue paper is covered with the visible irregular pattern of discrete coloured lotion spots containing a dye. Surprisingly, it has been found that this irregular pattern essentially does not reduce ply adhesion as generated by known embossing techniques.

The individual lotion spots preferably have an area of 0.01 to 30 mm², more preferably 0.02 to 15 mm², even more preferably 0.02 to 10 mm², in particular 0.02 to 6 mm².

The average area covered by the individual lotion spots ranges from 0.1 to 1 mm², preferably from 0.2 to 0.8 mm², in particular from 0.3 to 0.5 mm². The standard deviation from this mean value is preferably relatively low, i.e. from 0.4 to 0.8, more preferably from 0.5 to 0.7.

In first tests it has been confirmed that a lotioned tissue paper having the above (preferred) features was particularly well accepted in terms of aesthetic appearance.

The desired irregular pattern can be achieved with known lotions containing a dye in a suitable amount, preferably 0,1 to 20 wt.% based on the total lotion composition including the dye.

Suitable lotions may be selected from known water-free, water-in-oil (W/O), oil-in-water (O/W) and aqueous homogenous lotions. However, it is preferred to use aqueous, homogenous (single phase) lotions in combination with at least one water-soluble or water-dispersible dye. The most preferred mixing ratios dye/lotion range from about 1/100 to 10/100 based on the weight.

Furthermore, it is preferred to use lotions having a relatively low dynamic viscosity, preferably less than 500 mPas, more preferably less than 200 mPas, in particular less than 100 mPas, measured according to ASTM D2669 at 20°C. It is however also possible to use lotions having a higher viscosity at 20°C provided that these are heated prior to the application.

A preferred lotion comprises
- from 4.8 to 95 wt% water (preferably 10 to 50 wt. %, in particular 15 to 40 wt.%),
- 95 to 4.8 wt% of at least humidity regulator and
- from 0.1 to 20 wt% (preferably 0,5 to 15 wt.%, in particular 1 to 10 weight%) of at least one dye.

Other known lotion ingredients may be optionally used in amounts of up to 25 wt%, preferably 0.1 to 20, in particular 0.5 to 10 wt%. These optional ingredients include, but are not limited to skin care actives, preservatives, cosmetically acceptable surfactants, for instance the nonionic surfactants described in WO 98/41687, water-soluble or -dispersible polysiloxanes or other water-soluble or -dispersible oily agents which are known to have a skin protecting and/or tissue softening effect.

The skin care ingredients preferably have skin soothing and/or anti-inflammatory property. These include, but are not limited to skin care formulations based on vitamins or plant extracts, such as horse chestnut extract, birch extract, calendula, arnica extract, camomile or α-bisabolol, azulen, extract of rose of Sharon or St. Wort, teatree oil, cucumber, aloe vera, hops, allantoine, or hamamelis and lime tree which is known for its astringent and healing action. Pro-vitamin B5 (D-Panthenol) is also particularly well suited since it also serves as a moisturizing agent. Bisabolol and azulen provide a perceptible effect in amounts from 0.5 wt% to 1 wt% of the total composition. Other active ingredients that can be incorporated in the lotion composition include glycyrrhetinic acid, the active agent from liquorice, which has bacteriostatic and anti-inflammatory (antalgic) properties, as well as its salts and phytosterol (including ethoxylated ones) available under the trade name "Generol" from Henkel KgaA, that is fabricated from soya oil and also has anti-inflammatory action (see Parfümerie and Kosmetik 75 (1994) 775, R. Wachter, B. Salka and A. Magnet, "Phytosterole-pflanzliche Wirkstoffe in der Kosmetik"). These additional substances are preferably present in the composition in amounts between about 1 weight percent and about 20 weight percent of the total composition.

The amount of humidity regulators preferably ranges from 40 to 90 wt%, in particular from 50 to 80 wt%, based on the total lotion weight including the dye.

The above humidity regulator is preferably a polyol. It is preferred to use polyols being liquid at room temperature (20°C). Preferred humidity regulators can be selected from glycerol, polyalkylene glycol, such as diethylenglycol, propylene glycol or 1,3- butylene glycol, sugar alcohols and/or low molecular weight polyethylene glycols. It is preferred to use a combination of glycerol and propylene glycol.

A particularly preferred lotion is commercially available from Condea Servon BV, Delden, The Netherlands (now "Sasol Ltd., South Africa") under the trade name "Servon GMN 700E" and contains 45 wt% glycerol, 25 wt% water and 30 wt% propylenglycol. 3 wt% linden tree extract may be added thereto as skin care active.

Other suitable lotions are Servosoft XEG from Condea Servon and the Nopcosoft series from Nopco, Drammen, Norway.

Any dye can be included in the lotion composition as long as it results in clearly visible distinct lotion spots after the application on tissue paper and shows a strong affinity to the (cellulosic) fibers of the tissue paper. Preferably, water- soluble or water-dispersible dyes are used. Suitable dyes can be selected from basic, acid, or direct dyes. Among these, azo dyes are particularly preferred. Cellulose fibers as typically used in the tissue production often have negatively charged fiber surfaces, which can be combined with basic dye stuffs. However, it has been found that direct (substantive) dyestuffs are particularly suitable to obtain clearly visible lotion spots. The majority of direct dyes is based on water-soluble or water-dispersible azo dyes. In many cases water solubility is imparted by one or more sulfo groups. Direct azo dyes can be exemplified by disazo, trisazo and higher azo dyes. Their capacity to adhere to cellulosic webs increases with the number of planar conjugated n-double bond systems.

A particularly preferred azo direct dye is direct violet (C.I.: 27905 Dir. Violet. 51). This direct dye is marketed by Ciba-Geigy under the trade name "Pergasol Violet RA flüssig". Other direct dyes, such as direct blue 5BN LIQ (C.I.: 24400 Direct Blue 15), direct brown GSV LIQ (C.I.: 35005 Dir. Brown 44), chrysophenine G (C.I.: 24895 Dir. Yellow 12), direct yellow MV LIQ (C.I.: 40000 Dir. Yellow 11), direct black L LIQ or direct red MV (C.I.: 28160, Dir. Red 81) may also be used. Generally, it is preferred to use more intense colours, such as blue, red, green, brown, black or violet in order to achieve the necessary contrast to the white tissue paper.

The application amount of the dye-containing lotion is preferably 1 to 15%, more preferably 1.5 to 10 wt%, in particular 2 to 3% based on the weight on dry tissue paper.

In the following, tissue paper and its manufacture will be further explained. This description is applicable to the present invention.

A tissue paper is defined as a soft absorbent paper having a low basis weight. One generally selects a basis weight of 8 to 30 g/m², especially 10 to 25 g/m² per ply. The total basis weight of multiple-ply tissue products is preferably equal to a maximum of 70 g/m², more preferably to a maximum of 65 g/m². Its density is typically below 0.6 g/cm³, preferably below 0.30 g/cm³ and more preferably between 0.08 and 0.20 g/cm³.

The production of tissue is distinguished from paper production by its lower basis weight and its much higher tensile energy absorption index (see DIN EN 12625-4 and DIN EN 12625-5). Paper and tissue paper also differ in general with regard to the modulus of elasticity that characterizes the stress-strain properties of these planar products as a material parameter.

A tissue's high tensile energy absorption index results from the outer or inner creping. The former is produced by compression of the paper web adhering to a dry cylinder as a result of the action of a crepe doctor or in the latter instance as a result of a difference in speed ("rush transfer") between two wires ("fabrics"). This causes the still moist, plastically deformable paper web to be internally broken up by compression and shearing, thereby rendering it more stretchable under load than an uncreped paper.

Moist tissue paper webs are usually dried by the so-called Yankee drying, the through air drying (TAD) or the impulse drying method.

Preferably, the dye-containing lotion is applied on a dry tissue paper as obtained by the processes as described hereinbefore. This tissue paper is a creped or so-called "uncreped" tissue paper as obtainable by the already mentioned rush transfer process. Preferably, the tissue web to be lotioned also has been subjected to a through air drying step (TAD).

The fibers contained in the tissue paper are mainly cellulosic fibres, such as pulp fibers from chemical pulp (e.g. Kraft sulfite and sulfate pulps), mechanical pulp (e.g. ground wood), thermo mechanical pulp, chemo-mechanical pulp and/or chemo-thermo mechanical pulp (CTMP). Pulps derived from both deciduous (hardwood) and coniferous (softwood) can be used. The fibers may also be or include recycled fibers, which may contain any or all of the above categories. The fibers can be treated with additives - such as fillers, softeners, such as quaternary ammonium compounds and binders, such as conventional dry-strength agents or wet-strength agents used to facilitate the original paper making or to adjust the properties thereof. The tissue paper may also contain other types of fibers, e.g. regenerated cellulosic fibres or synthetic fibers enhancing, for instance, strength, absorption, smoothness or softness of the paper.

The tissue manufacture process essentially comprises
- a: forming that includes the headbox and the wire portion,
- b: the drying portion (TAD (through air drying)) or conventional drying on the yankee cylinder) that also usually includes the crepe process essential for tissues,
- c: typically the monitoring and winding area.

Paper can be formed by placing ("wet-laying")the fibers, in an oriented or random manner, on one or between two continuously revolving wires of a paper making machine while simultaneously removing the main quantity of water of dilution until dry-solids contents of usually between 12 and 35 % are obtained.

Drying the formed primary fibrous web occurs in one or more steps by mechanical and thermal means until a final dry-solids content of usually about 93 to 97 %. In the case of tissue making, this stage is followed by the crepe process which crucially influences the properties of the finished tissue product in conventional processes. The conventional dry crepe process involves creping on a usually 4.5 to 6 m diameter drying cylinder, the so-called yankee cylinder, by means of a crepe doctor with the aforementioned final dry-solids content of the raw tissue paper (wet creping can be used if lower demands are made of the tissue quality). The creped, finally dry raw tissue paper (raw tissue) is then available for further processing into the paper product or tissue paper product according to the invention.

Instead of the conventional tissue making process described above, the invention gives preference to the use of a modified technique in which an improvement in specific volume is achieved by a special kind of drying within process section b and in this way an improvement in the bulk softness of the thus made tissue paper is achieved. This process, which exists in a variety of subtypes, is termed the TAD (through air drying) technique. It is characterized by the fact that the "primary" fibrous web (like a nonwoven) that leaves the sheet making stage is pre-dried to a dry-solids content of about 80% before final contact drying on the yankee cylinder by blowing hot air through the fibrous web. The fibrous web is supported by an air-permeable wire or belt and during its transport is guided over the surface of an air-permeable rotating cylinder drum. Structuring the supporting wire or belt makes it possible to produce any pattern of compressed zones broken up by deformation in the moist state, resulting in increased mean specific volumes and consequently leading to an increase in bulk softness without decisively decreasing the strength of the fibrous web .

Another possible influence on the softness and strength of the raw tissue lies in the production of a layering in which the primary fibrous web to be formed is built up by a specially constructed headbox in the form of physically different layers of fibrous material, these layers being jointly supplied as a pulp strand to the sheet making stage.

When processing the raw fibrous web or raw tissue paper into the final product ("converting"), the following procedural steps are normally used individually or in combination: cutting to size (longitudinally and/or cross cutting), producing a plurality of plies, producing mechanical (embossing, knurling) and/or chemical (adhesive) ply adhesion, volumetric and structural embossing, folding, imprinting, perforating, application of lotions, smoothing, stacking, rolling up.

To produce multi-ply tissue paper products, such as handkerchiefs, toilet paper, towels or kitchen towels, an intermediate step preferably occurs with so-called doubling in which the raw tissue in the finished product's desired number of plies is usually gathered on a common multiply master roll.

The processing step from the raw tissue that has already been optionally wound up in several plies to the finished tissue product occurs in processing machines which include operations such as repeated smoothing of the tissue, edge embossing, to an extent combined with full area and/or local application of adhesive to produce ply adhesion of the individual plies (raw tissue) to be combined together, as well as longitudinal cut, folding, cross cut, placement and bringing together a plurality of individual tissues and their packaging as well as bringing them together to form larger surrounding packaging or bundles. The individual paper ply webs can also be pre-embossed and then combined in a roll gap according to the foot-to-foot or nested methods.

Generally it is possible to apply the coloured lotion at any suitable point in time and place after drying (and creping) on the Yankee cylinder in a paper machine. Thus, the application may take place during the doubling process of single tissue paper webs to multiply webs, as a process step in the converting machine, or as an extra step between doubling and converting. It is preferred to carry out the lotion application during the doubling or converting process, in particular before any calendaring steps are conducted.

The present invention can be employed for all different types of tissue paper products known in the art, such as handkerchiefs, facials, toilet paper or napkins.

According to the present invention, it is preferred to treat tissue paper webs as used in the manufacture of handkerchiefs or toilet tissue with the coloured lotion. The resulting tissue paper product is preferably constituted by 2-4 tissue plies. Each ply may consist of several layers, preferably two or three layers.

In the manufacture of multi-ply tissue products it is possible to treat the tissue webs that will constitute the outer product surfaces before or after they are combined and cut-to size. The lotion treatment may also occur after cutting to the final size of the commercial product.

The desired pattern of lotion spots is preferably obtained by spray devices. For this purpose, the application conditions must be chosen in a manner resulting in the target pattern of irregular discrete lotion spots. Nozzle-based or rotor-based spray devices are for instance suitable to practise the present invention. Examples for usable devices involve two component jets (nozzles) or the "rotor damping system compact II" which is available from WEKO Biel AG, Switzerland.

The WEKO rotor damping system compact II comprises two rotor carriers each having 12 rotors of the type 1/1 (normal rotor type), an operating unit, a digitacho with pulse generator, a pressure sensor, a constrictor hose (white, perimeter of 0,54mm) and a container as lotion supply unit. Via a belt and pulley an electrical motor drives the two rotor carriers, said pulley being available in two sizes (standard or bigger).

Depending on the width of the tissue web to be treated, the number of liquid-spraying rotors is selected. The other rotors then must be covered. To achieve different rotation speeds two pulleys having different sizes can be used to drive the belt of the rotors. Lotion and dye can be mixed in the container and then pumped to the rotor carriers. Centrifugal forces then make the dye-containing lotion leave the rotors and transfer the same to the tissue web in an irregular pattern. If only one tissue surface is to be lotioned, one rotor carrier suffices. Otherwise, both rotor carriers are used.

The shape and the dimension of the lotions spots can be suitably adjusted by adapting the lotion viscosity, the application amount of the lotion, the type and amount of the dye and/or the application equipment (e.g., in the case of two component jets, process conditions like air supply, lotion supply, nozzle type or, for rotor systems, the rotation per minute, the lotion supply and the type of rotors).

According to a second embodiment of the present invention, water is used instead of lotion to generate an irregular pattern of discrete dye spots on the tissue paper. Thereby, the same aesthetically appealing effect can be achieved. The above description is fully applicable to this embodiment apart from the necessary exchange of the dye-containing lotion by an aqueous solution of this dye, which preferably contains the same in an amount of 0.1 to 10 wt%, preferably 0.5 to 15 wt%, in particular 1 to 10 wt.%. More preferably, this aqueous solution consists only of water and dye.

The present invention is now described in further detail by two examples.

### Example 1

Dry and creped TAD tissue paper was lotioned in a PCMC converting line (type 12H, manufactured by Paper Converting Machine Company Ltd.) for handkerchiefs. This converting line comprised a pivot unwind, five web guide system, a mount hope roll, a calendar unit, an embossing unit to generate ply adhesion, preferably of SSE-type, a slitter, a device for folding in machine direction, a cross cutting device, a device for folding in cross direction and a device for stacking 10 handkerchiefs which then can be packaged. In the SSE system the paper plies are embossed on a hard roll with hard protrusions and then led through a nip between the hard embossing roll and another hard roll with a flat surface. In this nip the plies are mechanically bonded to each other.

A WEKO "rotor damping system compact II" as already described was arranged within this converting line after the web control system thus leading to the following sequence of processing steps: unwind stand - web control system - WEKO "rotor damping system compact II" - calendar unit - embossing unit.

Typical process conditions for the converting line involve a calendar pressure of 15-20 bar and an embossing pressure of 40 bar.

As first step of the inventive application process, the lotion was mixed with the dye in the container of the WEKO compact II application system. 50 litre of Servon GMN 700E (45 wt% glycerol, 25 wt% water, and 30 wt% propylenglycol, density 1,13 kg/l; available from Condea Servon BV) and 500 ml Pergasol Violet RA flüssig (azo dye, C.I., Dir. Violett 51, No. 27905, available for instance from Ciba-Geigy) were stirred for several minutes and then left standing over night. On the next morning, a homogenous solution mixture was obtained.

The digitacho of the WEKO system is then installed in the handkerchief line to determine the machine speed. The generated electrical signal was delivered to the operator unit to calculate automatically the right lotion application amount. Then, the dye-containing lotion is pumped from the container to the rotor carriers. The liquid flow is split in 12 parts so that each rotor receives the same amount of liquid. The liquid was applied to the middle of the rotors. Due to the rotation the liquid is ejected from the rotors thereby forming small droplets, which had the appearance of an aerosol. In this manner the droplets are transferred to the tissue web and coloured with an irregular (random) pattern. The distance between the circumference of the rotor carriers and the tissue web was approximately 150mm.

A four ply tissue web was led between the two carriers of the Weko system. Thereby both outer sides of the outer plyes received the irregular pattern of dye-containing lotion. The four ply tissue paper web had a basis weight of 15 g/m² per ply.

The four-ply tissue paper web carrying coloured lotions spots on both outer surfaces was then converted to conventional handkerchiefs and included in a conventional polymer film-based package. This package can be provided with a closure tape, which has the same colour as the lotion spots which creates a particularly pleasing optical effect.

In order to study the effect of application amount and rotational speed on the pattern generated, four tests were conducted wherein the application amount (2% or 3%, based on the weight of the dry tissue paper web) and the rotational speed were varied. To achieve different rotation speeds, two pulleys having different diameters (big: 75mm, small: 42mm) were used to drive the belt of the rotors.

The resulting lotioned tissue papers were examined with an optical picture analysis system (Optimas 6.1. available from Stemmer Imaging GmbH, Puchheim Germany) to determine the spot area, the mean value thereof and the standard deviation as well as the tissue paper area covered by the spots. The results are shown in the following table

| Example | Amount of lotion (wt%) | Rotat. speed | Min. area of spots /mm² | Max. area of spots /mm2 | Mean value /mm² | Standard deviation | Covered area (%) |
|---|---|---|---|---|---|---|---|
| A | 2 | low | 0,02 | 8 | 0,4 | 0,6 | 14 |
| B | 2 | high | 0, 02 | 9, 8 | 0,35 | 0,67 | 14 |
| C | 3 | low | 0,02 | 4,4 | 0,4 | 0,55 | 14 |
| D | 3 | high | 0,02 | 7 | 0,33 | 0,55 | 13 |

The comparison of these four samples further revealed the following tendencies. The use of smaller pulleys (lower rotational speed) generally seems to create a higher number of bigger spots (higher mean value) which however show a relatively small standard deviation. Bigger pulleys (higher rotational speeds), on the other hand, seem to result in a higher number of spots altogether which are however smaller since the covered area does not essentially change.

Among the four samples, sample C showed the aesthetically most pleasing appearance. The same is also shown as figure 1.

## Claims

1. Tissue paper carrying on at least one outer surface a visible irregular pattern of discrete coloured spots.

2. Tissue paper according to claim 1 wherein the irregular pattern of discrete coloured spots is obtainable by applying an aqueous solution of a dye.

3. Tissue paper according to claim 1 wherein the tissue paper is lotioned and the irregular pattern of discrete coloured spots is obtainable by applying a lotion containing a dye.

4. Tissue according to any of claims 1 to 3 wherein the coloured spots cover an area of 1 to 70 % Of the treated surface.

5. Tissue according to any of claims 1 to 4 wherein the lotion spots have an area of 0,01 to 30 mm².

6. Tissue according to any of claims 1 to 5 wherein the average area of the lotion spots ranges from 0,1 to 1 mm².

7. Tissue according to claim 6 wherein the standard deviation is from 0,4 to 0,8.

8. Tissue according to any of the preceding claims wherein the irregular pattern is obtainable by applying the dye-containing mixture, in particular lotion with a spraying device.

9. Tissue according to any of claims 3 to 8 wherein the lotion comprises from 4,8 to 95 weight% water, from 95 to 4,8 weight % of at least one humidity regulator, preferably polyols and from 0,1 to 20 weight % of at least one dye.

10. Tissue according to any of the preceding claims wherein the dye is a direct azo dye.

11. Lotioned tissue according to any of claims 3 to 10 wherein the lotion is added to the tissue in an amount of 1 to 15 % based on the basis weight of the dry tissue.

12. Process for the manufacture of a tissue paper as defined in any of claims 1 to 11 comprising the step of applying on at least one outer surface of the tissue a visible irregular pattern of discrete coloured spots.

13. Process according to claims 12 wherein an aqueous solution of a dye is applied to the tissue in a manner generating an irregular pattern of discrete coloured spots.

14. Process according to claims 12 wherein a lotion containing a dye is applied to the tissue in a manner generating an irregular pattern of discrete coloured spots.

15. Process according to any of claims 12 to 14 wherein a spraying device is used for applying the discrete coloured spots.
